# EUROPEAN PATENT SPECIFICATION

(11) **EP 2 344 166 B1**
(45) Date of publication and mention of the grant of the patent: **05.12.2012**
(21) Application number: 09736868.2
(22) Date of filing: 16.09.2009
(51) Int. Cl.: A61K 31/70, A61K 33/06, A61K 33/08, A61K 31/683, A61K 31/685, A61K 31/688

(54) **PHARMACEUTICAL COMPOSITION FOR THE TREATMENT OF GASTROINTESTINAL IRRITATION DISORDERS**
PHARMAZEUTISCHE ZUSAMMENSETZUNG ZUR BEHANDLUNG VON GASTROINTESTINALEN REIZUNGEN
COMPOSITION PHARMACEUTIQUE POUR TRAITEMENT DE TROUBLES D'IRRITATION GASTRO-INTESTINALE

(30) Priority: 17.09.2008 EP 08425607
(43) Date of publication of application: 20.07.2011
(73) Proprietor: Giuliani S.p.A., 20129 Milano (IT)
(72) Inventor: BELLINVIA, Salvatore, Pordenone 33170 (IT); MARTELLI, Laura, I-35126 Padova (IT); MARTELLI, Mario, Padova 35126 (IT)
(74) Representative: Appoloni, Romano
(86) International application number: PCT/EP2009/061997
(87) International publication number: WO 2010/031785

(56) References cited:
- EP-A- 0 092 121
- GB-A- 2 195 890
- US-A- 4 064 234
- US-A- 4 542 019
- US-A- 4 609 543
- US-A- 4 918 063
- US-B1- 7 226 916
- KIM J S: "Antifatigue health supplementary food" WPI/THOMSON,, vol. 2002, no. 7, 4 July 2001 (2001-07-04), XP002513250
- SZELENYI I ET AL: "Cytoprotective role of gastric surfactant in the ethanol-produced gastric mucosal injury of the rat." PHARMACOLOGY 1986, vol. 33, no. 4, 1986, pages 199-205, XP009111604 ISSN: 0031-7012
- BENGMARK S ET AL: "IMMUNONUTRITION: ROLE OF BIOSURFACTANTS, FIBER, AND PROBIOTIC BACTERIA" NUTRITION, ELSEVIER INC, US, vol. 14, no. 7/08, 1 January 1998 (1998-01-01), pages 585-594, XP001172824 ISSN: 0899-9007

## Description

The present invention relates to pharmaceutical compositions comprising an active principle for the treatment of disorders related to gastrointestinal irritation such as gastric ulcers, duodenal ulcers, acute gastritis, symptomatic chronic gastritis, gastropathy related to anti-inflammatories, reflux esophagitis, such as primarily Sucralfate.

Sucralfate, being a complex of saccharose sulphate with aluminium hydroxide, i.e. hexadeca-µ-hydroxytetracosahydroxy[µ₈-[1,3,4,6-tetra-O-sulfo-β-Dfructofuranosyl-α-D-glucopyranoside tetrakis(hydrogensulfato)8-)]]hexadecaaluminum, is a known active principle used for the treatment of pathologies related to gastrointestinal irritation, including those involving gastric mucosa lesions and ulcers able to cause bleeding. It is indicated particularly in the case of gastric ulcers, duodenal ulcers, acute gastritis, symptomatic chronic gastritis, gastropathy related to NSAIDs (non-steroidal anti-inflammatories), reflux esophagitis.

Physiological irritants conducive to these pathologies include gastric acid, bile acids, their salts and pepsin. Sucralfate is able to counteract intestinal irritants such as bile acids and salts [Tanghöj H et al. Gastroenterology. 1985; 88: 1699], various pepsinogens [Samloff IM Am. J. Med. 1985; 79 (2c): 15-18] and the effects of gastric acidity, by rendering the epithelium resistant to acid [Orlando RC. Gastroenterology. 1987; 93: 352-61]. These effects are best achieved if Sucralfate is consumed in a pharmaceutical form that allows maximum dispersion of the active principle in the entire gastric system within the shortest time. Sucralfate is consumed *per os,* the pharmaceutical form being prepared for proper dispersion in a liquid formulation which transits within the oral cavity: it is essential therefore that the preparation has good palatability. Sucralfate, however, when dispersed in any vehicle, presents an unpleasant sensation in the oral cavity, similar to a dry powder adhering to the tongue in the case of a dispersed solid powder, or an astringent sensation especially in the case of wet gels.

This flaw results in a serious lack of compliance by the treated individuals, such as to compromise regular consumption and hence therapy effectiveness.

According to the present invention it has been surprisingly found that Sucralfate, in whatever pharmaceutical formulation deemed suitable for its oral use, acquires a considerable palatability improvement, i.e. without the dry tongue or astringent effect, when formulated in the presence of phospholipids.

According to the present invention it has also been surprisingly found that Sucralfate, when formulated in this manner with phospholipids for oral use, is able to perform its pharmacological activity in an enhanced manner according to a synergistic pattern. This, among other things, enables the same therapeutic effect to be produced by using a lower dose of the Sucralfate active principle than in the known art.

Phospholipids are known as being protective of the esophageal mucosa especially in bile acid reflux [Gabor E. et al. World J. Gastroenterol. 2006; 12(2): 271-279], and for enhancing the therapeutic activity of NSAIDS (non-steroidal anti-inflammatories) and reducing their effects as gastric irritants (PCT/US97/16994 or WO 98/13073), but their use is not associated with Sucralfate.

Their palatability and anti-astringency effect on Sucralfate, as well as the synergistically enhanced pharmacological activity compared to Sucralfate alone, is therefore new and not predictable from the prior art.

The above can be applied to other active principles, analogous to Sucralfate, for treating gastrointestinal irritation disorders, such as Al(OH)₃, Mg(OH)₂, MgO, magaldrate, as such or in a mixture.

The present invention relates to a pharmaceutical composition comprising sucralfate as active principle for the treatment of gastrointestinal irritation disorders such as gastric ulcers, duodenal ulcers, acute gastritis, symptomatic chronic gastritis, gastropathy related to anti-inflammatories, reflux esophagitis characterized by comprising one or more phospholipids.

The present invention also relates to the use of phospholipids in combination with sucralfate for improving its palatability and to counter its tongue drying and astringent effect, when administrated orally.

The present invention also relates to the use of phospholipids in combination with sucralfate for the preparation of a composition to be administered orally for the treatment of gastrointestinal irritation disorders and to synergistically improve its anti-ulcer effect.

Said phospholipids are preferably chosen from the following: phosphatidylcholine, phosphatidylserine, phosphatidylethanolamine, phosphatidylinositol, dipalmitoylphosphatidylcholine, lysophospholipids, zwitterionic phospholipids, sphingomyelins or mixtures thereof.

Additional phospholipids suitable for the invention are dilinoleoylphosphatidylcholine (DLL-PC), phospholipids derived from eggs (Egg-PC) or from milk.

In the composition of the invention high concentrations of polar phospholipids are preferable, i.e. phospholipids substantially insoluble in acetone (see for example, Lecithin-Properties and Applications, page 9. Lucas Meyer (GmbH and Co) Ausschlayer Elbdeich 62 D-2000 Hamburg 26; Form and function of phospholipids Ed. G. B. Ansell, R. M. C. Dawson and J. M. Hawthorne, Elsevier-Amsterdam-London-New York 1973, page 45).

A composition according to the invention preferably contains sucralfate and phospholipids in a weight ratio of about 1:0.1 to about 1:20.

A composition according to the invention more preferably contains sucralfate and phospholipids in a weight ratio of about 1:0.5 to about 1:2.

A composition according to the invention even more preferably contains sucralfate and phospholipids in a weight ratio of 1:1.

In one embodiment of the invention said phospholipids are in admixture with fatty acids, preferably unsaturated and polyunsaturated. Preferably mono- and polyunsaturated fatty acids are present in a quantity greater than 10% of total fatty acids.

In particular, a combination of Sucralfate with polar phospholipids is provided, characterized by a high polyunsaturated fatty acid content of, for example, between 62 and 80%.

A composition of the invention can also comprise synthetic phospholipids or other lipids such as triglycerides.

Suitable phospholipids for a composition of the invention are also mixtures present in commercial products such as EPICURON 130™ having a greater than 97% degree of acetone insolubility and therefore being polar, comprising from 30 to 35% by weight of phosphatidylcholine, from 15 to 20% by weight of phosphatidylethanolamine, and from 8 to 13% by weight of phosphatidylinositol.

Another suitable commercial product comprising a mixture of phospholipids with 60-68% polyunsaturated fatty acids is for example Essentiale Forte N™.

A composition of the invention can also comprise additional substances with antacid activity such as aluminium hydroxide, magnesium hydroxide, MgO, carbonates or bicarbonates of alkali or alkaline-earth metals, magaldrates, simethicone, salts of alginic acid which enable formation of gelatinous foams, hydrolyzed or non-hydrolyzed chitins, saccharide polymers such as glucomannans or PEG, cimetidine and similar H2 blockers, misoprostol and proton pump inhibitors (PPI). Active principles which augment the tone of the esophageal sphincter and accelerate gastric emptying can also be added to the composition of the invention, these being conditions which can reduce contact between the esophageal mucosa and gastric irritants.

The composition for oral use of the invention is prepared in a suitable pharmaceutical form such as a solid powder, or a gel or in liquid form.

Finally, the composition of the invention can comprise pharmaceutically acceptable excipients such as solvents, dispersants, antioxidants, carriers, coatings.

The following examples illustrate the invention without in any way limiting its scope.

### Examples of formulations

### Example 1

| | |
|---|---|
| Sucralfate | 2g |
| EPICURON 130 | 2g |
| Sorbitol 70% | 2g |
| Sodium benzoate | 0.02g |
| Methyl paraben | 0.0206g |
| Propyl paraben | 0.0024g |
| Aspartame | 0.02g |
| Flavour | 0.015g |
| Water | q.b. to 10ml |

### Example 2

### Liquid suspension (for 1 dose) containing:

| | |
|---|---|
| Sucralfate | 1.0g |
| Magaldrate | 0.5g |
| EPICURON 130 | 2.0g |
| Apple flavour | 0.015g |
| Aspartame | 0.02g |
| Water | 15ml |

This composition can be dispersed in fruit juices or other drinks.

### Example 3

### Liquid suspension (for 1 dose) containing:

| | |
|---|---|
| Sucralfate | 1.0g |
| EPICURON 130 | 2.0g |
| Apple flavour | 0.015g |
| Aspartame | 0.02g |
| Mg(OH)₂ | 0.3g |
| Water | 15ml |

This composition can be dispersed in fruit juices or other drinks.

### Example 4

### Liquid suspension (for 1 dose) containing:

| | |
|---|---|
| Sucralfate | 0.5g |
| EPICURON 130 | 0.8g |
| Ca(CO)₃ | 0.2g |
| K alginate | 1.0g |
| Basic Mg carbonate | 0.3g |
| Water | 0.25g |
| Suspending agent PEG 4000 | 0.3g |
| Flavours and sweeteners | q.b. |

This composition can be dispersed in fruit juices or other drinks.

### Example 5

### Liquid suspension (for 1 dose) containing:

| | |
|---|---|
| MgO | 0.3g |
| EPICURON 130 | 1.0g |
| Apple flavour | 0.015g |
| Aspartame | 0.02g |
| Water | 15ml |

This composition can be dispersed in fruit juices or other drinks.

### Example 6

### Liquid suspension (for one dose) containing:

| | |
|---|---|
| Magaldrate | 0.4g |
| EPICURON 130 | 1.0 g |
| Apple flavour | 0.015 g |
| Aspartame | 0.02 g |
| Water | 15 ml |

This composition can be dispersed in fruit juices or other drinks.

### Example 7

### Liquid suspension (for one dose) containing:

| | |
|---|---|
| Al(OH)₃ | 0.3 g |
| EPICURON 130 | 1.0 g |
| Apple flavour | 0.015 g |
| Aspartame | 0.02 g |
| Water | 15 ml |

The following examples illustrate the experimental use *in vivo* of the compositions of the invention without in any way limiting its scope.

The commercial product EPICURON 130™ cited therein, comprises from 30 to 35% by weight of phosphatidylcholine, from 15 to 20% by weight of phosphatidylethanolamine and from 8 to 13% by weight of phosphatidylinositol. The activity of Sucralfate+EPICURON 130 compositions was tested on models of ulcers induced by ethanol, NSAIDs (acetyl salicylic acid or aspirin) or *Helicobacter pylori* (HP), either as a preventative or as a treatment for the actual gastric damage. In addition, the activity of Sucralfate+EPICURON 130 compositions was tested on gastroesophageal regurgitation.

### Example 8: lesions induced by a NSAID (acetyl salicylic acid)

Rats weighing 205-215 g were allowed to fast overnight. The following day a dose of Sucralfate (300 mg/kg suspended in saline solution) and a dose of the composition of the invention comprising Sucralfate and EPICURON 130 in a mutual weight ratio of 1:1 (300 mg/kg suspended in saline solution) was administered by gavage one hour before ulcer induction by acetyl salicylic acid administration (150 mg/kg). The cytoprotective effect was evaluated 4 hours after administration of acetyl salicylic acid. Whereas the Sucralfate prevented gastric ulcer formation by 50% (in relation to a control, treated with saline solution) the Sucralfate+EPICURON 130 was 1.8-1.9 times more active, preventing ulcer formation by up to 90-95%. Evaluation of percentage ulcer repair was carried out with the method described by Pozzoli C. Naunyn-Schmiedeberg's Arch. Pharmacol. 2007, 374, 283-291.

### Example 9

A second experiment was conduced in accordance with Hardin CK [Am. Surg. 1987; 53(7):373-6], whereby aspirin (150 mg/kg) was administered followed, after 5 minutes, by Sucralfate (300 mg/kg suspended in saline solution) and Sucralfate + EPICURON 130 in a 1:1 ratio (300 mg/kg suspended in saline solution). After 4 hours, residual gastric damage was evaluated by the same method as aforesaid. In this case gastric ulcer repair effected by Sucralfate was equal to 30-35%, whereas Sucralfate+EPICURON 130 led to the repair of about 75-85% of the ulcer itself.

Sucralfate and Sucralfate+EPICURON 130 were shown to be less effective in repairing the ulcer than preventing its formation, confirming the results of Hardin CK [Am. Surg. 1987; 53(7):373-6], though Sucralfate+EPICURON 130 was 2.5-2.8 more effective than Sucralfate alone.

### Example 10: ethanol induced lesions

The sequence of operation was identical to the aforedescribed and the amounts of active principles used were also identical.

In this experiment Sucralfate and Sucralfate+EPICURON 130 were administered by gavage 15 minutes before administration of absolute ethanol (1 ml/rat). Evaluation of protection effected by Sucralfate and Sucralfate+EPICURON 130 was undertaken 1 hour after ethanol administration. The results showed that Sucralfate prevented ulcer formation by 50% while Sucralfate (300 ml/kg) + EPICURON 130 in a 1: 1 ratio (300 mg/kg) was 1.6-1.7 times more active, preventing ulcer formation by up to 80-85%.

An example of a clinical test is given hereinafter.

### Example 11: activity of Sucralfate+EPICURON 130 in a 1:1 ratio in ulcers induced by Helicobacter pylori (qualitative evaluation)

The results obtained with rats (see above) were used to evaluate the effects of the Sucralfate+EPICURON 130 preparation in a 1:1 ratio in ulcers induced by *Helicobacter pylori* (HP) in man.

Five volunteers who were carriers of HP infection, were subjected to gastroscopy (after giving their informed consent) in order to assess the condition of the gastrointestinal irritation before undergoing all other eradication therapies. After 1 week, having consumed 2 g of the Sucralfate+EPICURON 130 preparation in a 1: 1 ratio twice a day (in the morning and in the evening at bedtime), the gastroscopy was repeated and compared with the previous one.

The results showed a definite improvement in the condition of the gastric mucosa in all 5 cases assessed. The subjective qualitative assessment of well-being by the individual volunteers after consuming Sucralfate+EPICURON 130 in a 1:1 ratio was also positive, hence confirming the gastroscopy results.

### Example 12

The composition of example 1 above was tested on gastresophageal reflux.

Five volunteers who presented with symptoms of gastresophageal reflux disease and who had used other similar preparations, tested the composition of example 1 above and assessed the degree to which throat redness and irritation were alleviated.

The palatability of the preparation and degree of well-being afforded were also judged.

The results showed:
1) almost complete or complete disappearance of throat redness,
2) that palatability was, textually: "better than other previously tried preparations",
3) almost complete alleviation of gastrointestinal irritation (heartburn, sensation of postprandial heaviness) within 10 minutes of consuming the preparation.

### Comparison of palatability between Sucralfate and Sucralfate+EPICURON 130 1:1)

Seven volunteers were asked to select the more palatable product by comparing Sucralfate gel (Gastrogel™) and a composition of the invention comprising Sucralfate+EPICURON 130 (1:1)

The results are summarized in Table A:

**Table A**

| | volunteer | age | Selected composition |
|---|---|---|---|
| 1 | G.M. | 30 | Sucralfate+EPICURON 130 (1:1) |
| 2 | S.B. | 39 | Sucralfate+EPICURON 130 (1:1) |
| 3 | L.S. | 60 | Sucralfate+EPICURON 130 (1:1) |
| 4 | L.M. | 33 | Sucralfate+EPICURON 130 (1:1) |
| 5 | L.G. | 71 | Sucralfate+EPICURON 130 (1:1) |
| 6 | M.S. | 64 | Sucralfate+EPICURON 130 (1:1) |
| 7 | F.T. | 72 | Sucralfate+EPICURON 130 (1:1) |

All the volunteers therefore preferred the Sucralfate+EPICURON 130 product (1:1) of the invention.

As an integration to the preceding examples 8 to 10, the following further examples 13 to 15 relate to the same comparison of determined activities as reported above, but additionally report the activity determined after administration of EPICURON 130 alone.

It should be noted that in the preceding examples 8 to 10 and in the following examples 13 to 15, the administered dose of the composition of the invention, comprising Sucralfate and EPICURON 130 in a mutual weight ratio of 1:1 corresponding to an overall 300 mg/kg suspended in saline solution, includes 150 mg/kg of Sucralfate + 150 mg/kg of EPICURON 130, therefore half the amount of each component compared to when it is administered alone.

### Example 13: Prevention of lesions induced by NSAID (acetyl salicylic acid)

6 rats weighing 205-215 g were used for each test. These rats were kept under fasting conditions overnight. The following day a dose of Sucralfate alone, or EPICURON 130 alone or Sucralfate + EPICURON 130 (in a 1:1 weight ratio), all in an amount of 300 mg/kg, suspended in 2 ml of saline solution, were administered by gavage one hour before ulcer induction by acetyl salicylic acid administration (150 mg/kg - 1 ml in saline solution). The cytoprotective effect was evaluated 4 hours after administration of acetyl salicylic acid, by the method described by Pozzoli C. et al., Naunyn-Schmiedeberg's Arch. Pharmacol. 2007, 374, 283-291.

The control was treated with a 2 ml dose of saline solution.

The Sucralfate prevented ulcer formation by 50%, EPICURON 130 by 20-25% and Sucralfate + EPICURON 130 by 90-95%. Although the composition of the invention included half the amount of each component, the activity demonstrated was 1.3-1.4 times greater than the sum of the activities of the single components.

### Example 14: Repair of lesions induced by NSAID (acetyl salicylic acid)

The experimental conditions were as above.

This experiment was conducted according to Hardin C.K. Ann. Surg. 1987, 53(7), 373-376.

The acetyl salicylic acid was administered first by gavage (150 mg/kg in 1 ml of saline solution) followed, after 5 minutes, by administration of Sucralfate alone or EPICURON 130 alone, or Sucralfate + EPICURON 130 in a 1:1 weight ratio (all administrations were in an amount of 300 mg/kg suspended in 2 ml of saline solution).

The saline solution formed the control.

After 4 hours, residual gastric damage was evaluated by the same method as aforedescribed.

Compared to the control, damage repair effected by Sucralfate was 30-35%, 15-20% by EPICURON 130 and 75-85% by Sucralfate + EPICURON 130. Although the composition of the invention included half the amount of each component, the activity demonstrated was 1.5-1.7 times greater than the sum of the activities of the single components.

Sucralfate, EPICURON 130 and Sucralfate + EPICURON 130 proved to be less active in repairing ulcers than their prevention, hence confirming the results of Hardin (see earlier).

### Example 15: Prevention of ethanol-induced ulcers

The sequence of operation was identical to the aforedescribed and the amounts of active principles used for the test were the same.

Sucralfate, EPICURON 130 and Sucralfate + EPICURON 130 (in a 1:1 weight ratio) were administered by gavage 15 minutes before administration of absolute ethanol in an amount of 1 ml/rat. The evaluation of protective activity was undertaken 1 hour after ethanol administration. The results showed that ethanol-induced damage repair effected by Sucralfate was 45-50%, 22-25% by EPICURON 130 and 80-85% by Sucralfate + EPICURON 130.

Although the composition of the invention included half the amount of each compound, it demonstrated an activity 1.1-1.2 times greater than the sum of the activities of the single components.

## Claims

1. Pharmaceutical composition comprising sucralfate as active principle for the treatment of gastrointestinal irritation disorders such as gastric ulcers, duodenal ulcers, acute gastritis, symptomatic chronic gastritis, gastropathy related to anti-inflammatories, reflux esophagitis, **characterized by** comprising one or more phospholipids.

2. Composition according to claim 1 **characterized in that** it comprises one or more additional substances with antacid activity chosen from: Al(OH)₃, Mg(OH)₂, MgO, magaldrate, carbonates or bicarbonates of alkali or alkaline-earth metals, simethicone, salts of alginic acid which enable formation of gelatinous foams, hydrolyzed or non-hydrolyzed chitins, saccharide polymers, glucomannans, PEG, cimetidine and similar H2 blockers, misoprostol and proton pump inhibitors (PPI).

3. Composition according to claim 1 **characterized in that** said phospholipids are chosen from the following: phosphatidylcholine, phosphatidylserine, phosphatidylethanolamine, phosphatidylinositol, dipalmitoylphosphatidylcholine, lysophospholipids, zwitterionic phospholipids, sphingomyelins or mixtures thereof.

4. Composition according to claim 1 **characterized in that** said phospholipids are polar.

5. Composition according to claim 4 **characterized in that** said polar phospholipids have a higher than 97% degree of acetone insolubility.

6. Composition according to claim 1 **characterized in that** said phospholipids are in mixture with fatty acids.

7. Composition according to claim 6 **characterized in that** said phospholipids are in mixture with polyunsaturated fatty acids.

8. Composition according to claim 1 **characterized by** containing sucralfate and phospholipids in a weight ratio from about 1:0.1 to about 1:20.

9. Composition according to claim 1 **characterized by** containing sucralfate and phospholipids in a weight ratio from about 1:0.5 to about 1:2.

10. Composition according to claim 1 **characterized by** containing sucralfate and phospholipids in a weight ratio of 1:1.

11. Use of sucralfate in combination with phospholipids for the preparation of a composition to be administered orally for the treatment of gastrointestinal irritation disorders such as gastric ulcers, duodenal ulcers, acute gastritis, symptomatic chronic gastritis, gastropathy related to anti-inflammatories, reflux esophagitis, for improving palatability.

12. Use according to claim 11 **characterized in that** sucralfate is utilized with one or more additional substances with antacid activity chosen from Al(OH)₃, Mg(OH)₂, MgO, magaldrate, carbonates or bicarbonates of alkali or alkaline-earth metals, simethicone, salts of alginic acid which enable formation of gelatinous foams, hydrolyzed or non-hydrolyzed chitins, saccharide polymers, glucomannans, PEG, cimetidine and similar H2 blockers, misoprostol and proton pump inhibitors (PPI).

13. Use according to claim 11, wherein the said composition is to be administered orally for the treatment of gastrointestinal irritation disorders and to synergistically improve the antiulcer activity of sucralfate.

14. Use according to claim 11 **characterized in that** said phospholipids are chosen from the following: phosphatidylcholine, phosphatidylserine, phosphatidylethanolamine, phosphatidylinositol, dipalmitoylphosphatidylcholine, lysophospholipids, zwitterionic phospholipids, sphingomyelins or mixtures thereof.

15. Use according to claim 11 **characterized in that** said phospholipids are polar.

16. Use according to claim 15 **characterized in that** said phospholipids have a higher than 97% degree of acetone insolubility.

17. Use according to claim 11 **characterized by** mixing sucralfate and phospholipids in a weight ratio from about 1:0.1 to about 1:20.

18. Use according to claim 11 **characterized by** mixing sucralfate and phospholipids in a weight ratio from about 1:0.5 to about 1:2.

19. Use according to claim 11 **characterized by** mixing sucralfate and phospholipids in a weight ratio of 1:1.

## Patentansprüche

1. Pharmazeutische Zusammensetzung, welche Sucralfat als einen aktiven Hauptbestandteil enthält, für die Behandlung von gastrointestinalen Reizungsstörungen, wie beispielsweise Magengeschwüren, Zwölffingerdarmgeschwüren, akuter Gastritis, symptomatischer chronischer Gastritis, Gastropathie verbunden mit entzündungshemmenden Mitteln, Refluxösophagitis, **dadurch gekennzeichnet, dass** diese ein oder mehr Phospholipide enthält.

2. Zusammensetzung nach Anspruch 1, **dadurch gekennzeichnet, dass** diese ein oder mehrere weitere Substanzen mit Antisäureaktivität enthält, welche ausgewählt sind aus: Al(OH)₃, Mg(OH)₂, MgO, Magaldrat, Carbonaten oder Bicarbonaten von Alkali- oder Erdalkalimetallen, Simeticon, Salzen von Algininsäure, welche die Ausbildung von gelatinösen Schäumen ermöglichen, hydrolysierten oder nicht-hydrolysierten Chitinen, Saccharidpolymeren, Glucomannaneb, PEG, Cimetidin und ähnlichen H2-Blockern, Misoprostol und Protonenpumpinhibitoren (PPI).

3. Zusammensetzung nach Anspruch 1, **dadurch gekennzeichnet, dass** die Phospholipide aus den folgenden ausgewählt sind:
Phosphatidylcholin, Phosphatidylserin, Phosphatidylethanolamin, Phosphatidylinosit, Dipalmitoylphosphatidylcholin, Lysophospholipiden, zwitterionischen Phospholipiden, Sphingomyelinen oder Mischungen hiervon.

4. Zusammensetzung nach Anspruch 1, **dadurch gekennzeichnet, dass** die Phospholipide polar sind.

5. Zusammensetzung nach Anspruch 4, **dadurch gekennzeichnet, dass** die polaren Phospholipide einen höheren Grad an Acetonunlöslichkeit als 97 % aufweisen.

6. Zusammensetzung nach Anspruch 1, **dadurch gekennzeichnet, dass** die Phospholipide in Mischung mit Fettsäuren vorliegen.

7. Zusammensetzung nach Anspruch 6, **dadurch gekennzeichnet, dass** die Phospholipide in Mischung mit polyungesättigten Fettsäuren vorliegen.

8. Zusammensetzung nach Anspruch 1, **dadurch gekennzeichnet, dass** diese Sucralfat und Phospholipide in einem Gewichtsverhältnis von ungefähr 1:0,1 bis ungefähr 1:20 enthält.

9. Zusammensetzung nach Anspruch 1, **dadurch gekennzeichnet, dass** diese Sucralfat und Phospholipide in einem Gewichtsverhältnis von ungefähr 1:0,5 bis ungefähr 1:2 enthält.

10. Zusammensetzung nach Anspruch 1, **dadurch gekennzeichnet, dass** diese Sucralfat und Phospholipide in einem Gewichtsverhältnis von 1:1 enthält.

11. Verwendung von Sucralfat in Mischung mit Phospholipiden für die Herstellung einer Zusammensetzung, welche oral zu verabreichen ist, für die Behandlung von gastrointestinalen Reizungsstörungen, wie beispielsweise Magengeschwüren, Zwölffingerdarmgeschwüren, akuter Gastritis, symptomatischer chronischer Gastritis, Gastropathie verbunden mit entzündungshemmenden Mitteln, Refluxösophagitis, um die Schmackhaftigkeit zu verbessern.

12. Verwendung nach Anspruch 11, **dadurch gekennzeichnet, dass** Sucralfat zusammen mit einer oder mehreren weiteren Substanzen mit Antisäureaktivät ausgewählt aus Al(OH)₃, Mg(OH)₂, MgO, Magaldrat, Carbonaten oder Bicarbonaten von Alkali- oder Erdalkalimetallen, Simeticon, Salzen von Algininsäure, welche die Ausbildung von gelatinösen Schäumen ermöglichen, hydrolysierten oder nicht-hydrolysierten Chitinen, Saccharidpolymeren, Glucomannaneb, PEG, Cimetidin und ähnlichen H2-Blockern, Misoprostol und Protonenpumpinhibitoren (PPI)verwendet wird.

13. Verwendung nach Anspruch 11, wobei die Zusammensetzung zur Behandlung von gastrointestinalen Reizungsstörungen und für die synergistische Verbesserung der Antigeschwüraktivität von Sucralfat oral zu applizieren ist.

14. Verwendung nach Anspruch 11, **dadurch gekennzeichnet, dass** die Phospholipide aus den folgenden ausgewählt werden:
Phosphatidylcholin, Phosphatidylserin, Phosphatidylethanolamin, Phosphatidylinosit, Dipalmitoylphosphatidylcholin, Lysophospholipiden, zwitterionischen Phospholipiden, Sphingomyelinen oder Mischungen hiervon.

15. Verwendung nach Anspruch 11, **dadurch gekennzeichnet, dass** die Phospholipide polar sind.

16. Verwendung nach Anspruch 15, **dadurch gekennzeichnet, dass** die Phospholipide einen höheren Grat an Acetonunlöslichkeit als 97 % aufweisen.

17. Verwendung nach Anspruch 11 **gekennzeichnet durch** Vermischen von Sucralfalt und Phospholipiden in einem Gewichtsverhältnis von ungefähr 1:0,1 1 bis ungefähr 1:20.

18. Verwendung nach Anspruch 11 **gekennzeichnet durch** Vermischen von Sucralfalt und Phospholipiden in einem Gewichtsverhältnis von ungefähr 1:0,5 bis ungefähr 1:2.

19. Verwendung nach Anspruch 11, **gekennzeichnet durch** Vermischen von Sucralfalt und Phospholipiden in einem Gewichtsverhältnis von 1:1.

## Revendications

1. Composition pharmaceutique comprenant du sucralfate en tant que principe actif, pour le traitement des troubles d'irritation gastro-intestinale, tels que les ulcères gastriques, les ulcères du duodénum, la gastrite aiguë, la gastrite chronique symptomatique, une gastropathie associée aux anti-inflammatoires, l'oesophagite peptique, **caractérisée en ce qu'**elle comprend un ou plusieurs phospholipides.

2. Composition selon la revendication 1, **caractérisée en ce qu'**elle comprend une ou plusieurs substances supplémentaires ayant une activité antiacide choisies parmi : Al(OH)₃, Mg(OH)₂, MgO, le magaldrate, les carbonates ou les bicarbonates des métaux alcalins ou alcalino-terreux, la siméthicone, les sels de l'acide alginique qui permettent la formation de mousses gélatineuses, les chitines hydrolysées ou non hydrolysées, les polymères saccharidiques, les glucomannanes, le PEG, la cimétidine et les bloqueurs H2 similaires, le misoprostol et les inhibiteurs de pompe à protons (PPI).

3. Composition selon la revendication 1, **caractérisée en ce que** lesdits phospholipides sont choisis parmi les suivants : la phosphatidylcholine, la phosphatidylsérine, la phosphatidyléthanolamine, le phosphatidylinositol, la dipalmitoylphosphatidylcholine, les lysophospholipides, les phospholipides zwitterioniques, les sphingomyélines ou leurs mélanges.

4. Composition selon la revendication 1, **caractérisée en ce que** lesdits phospholipides sont polaires.

5. Composition selon la revendication 4, **caractérisée en ce que** lesdits phospholipides polaires présentent un degré d'insolubilité dans l'acétone supérieur à 97 %.

6. Composition selon la revendication 1, **caractérisée en ce que** lesdits phospholipides sont en mélange avec des acides gras.

7. Composition selon la revendication 6, **caractérisée en ce que** lesdits phospholipides sont en mélange avec des acides gras polyinsaturés.

8. Composition selon la revendication 1, **caractérisée en ce qu'**elle contient du sucralfate et des phospholipides à un rapport en poids d'environ 1:0,1 à environ 1:20.

9. Composition selon la revendication 1, **caractérisée en ce qu'**elle contient du sucralfate et des phospholipides à un rapport en poids d'environ 1:0,5 à environ 1:2.

10. Composition selon la revendication 1, **caractérisée en ce qu'**elle contient du sucralfate et des phospholipides à un rapport en poids de 1:1.

11. Utilisation de sucralfate en combinaison avec des phospholipides pour la préparation d'une composition à administrer par voie orale pour le traitement des troubles d'irritation gastro-intestinale, tels que les ulcères gastriques, les ulcères du duodénum, la gastrite aiguë, la gastrite chronique symptomatique, une gastropathie associée aux anti-inflammatoires, l'oesophagite peptique, pour améliorer la palatabilité.

12. Utilisation selon la revendication 11, **caractérisée en ce que** le sucralfate est utilisé avec une ou plusieurs substances supplémentaires ayant une activité antiacide choisies parmi Al(OH)₃, Mg(OH)₂, MgO, le magaldrate, les carbonates ou les bicarbonates des métaux alcalins ou alcalino-terreux, la siméthicone, les sels de l'acide alginique qui permettent la formation de mousses gélatineuses, les chitines hydrolysées ou non hydrolysées, les polymères saccharidiques, les glucomannanes, le PEG, la cimétidine et les bloqueurs H2 similaires, le misoprostol et les inhibiteurs de pompe à protons (PPI).

13. Utilisation selon la revendication 11, dans laquelle ladite composition est à administrer par voie orale pour le traitement des troubles d'irritation gastro-intestinale et est destinée à améliorer de manière synergique l'activité antiulcéreuse du sucralfate.

14. Utilisation selon la revendication 11, **caractérisée en ce que** lesdits phospholipides sont choisis parmi les suivants : la phosphatidylcholine, la phosphatidylsérine, la phosphatidyléthanolamine, le phosphatidylinositol, la dipalmitoylphosphatidylcholine, les lysophospholipides, les phospholipides zwitterioniques, les sphingomyélines ou leurs mélanges.

15. Utilisation selon la revendication 11, **caractérisée en ce que** lesdits phospholipides sont polaires.

16. Utilisation selon la revendication 15, **caractérisée en ce que** lesdits phospholipides présentent un degré d'insolubilité dans l'acétone supérieur à 97 %.

17. Utilisation selon la revendication 11, **caractérisée par** le mélange du sucralfate et des phospholipides à un rapport en poids d'environ 1:0,1 à environ 1:20.

18. Utilisation selon la revendication 11, **caractérisée par** le mélange du sucralfate et des phospholipides à un rapport en poids d'environ 1:0,5 à environ 1:2.

19. Utilisation selon la revendication 11, **caractérisée par** le mélange du sucralfate et des phospholipides à un rapport en poids de 1:1.
